# EUROPEAN PATENT SPECIFICATION

(11) **EP 1 358 201 B1**
(45) Date of publication and mention of the grant of the patent: **07.10.2009**
(21) Application number: 02718087.6
(22) Date of filing: 04.02.2002
(51) Int. Cl.: C07J 41/00

(54) **A PROCESS FOR THE PREPARATION OF 3-GLUTAMIDO BILE ESTER DERIVATIVES USING N-PROTECTED METHYL PYROGLUTAMATE**
VERFAHREN ZUR HERSTELLUNG VON 3-GLUTAMID GALLENSÄUREDERIVATEN DURCH VERWENDUNG VON N-GESCHÜTZTEN METHYL-PYROGLUTAMAT
PROC D DE PR PARATION DE D RIV S D'ESTERS DE BILE 3-GLUTAMIDO AU MOYEN DE PYROGLUTAMATE DE METHYLE N-PROTEGE

(30) Priority: 05.02.2001 EP 01102490
(43) Date of publication of application: 05.11.2003
(73) Proprietor: BRACCO IMAGING S.p.A., 20134 Milano (IT)
(72) Inventor: BROCCHETTA, Marino, I-20134 Milano (IT); ANELLI, Pier, Lucio, I-20134 Milano (IT); MANFREDI, Giuseppe, I-20134 Milano (IT); VISIGALLI, Massimo, I-20134 Milano (IT); PALANO, Daniela, I-20134 Milano (IT); ALESSANDRONI, Laura, I-20134 Milano (IT)
(74) Representative: Macchetta, Francesco
(86) International application number: PCT/EP2002/001133
(87) International publication number: WO 2002/068449

(56) References cited:
- WO-A-00/38738
- KOTSUKI, HIYOSHIZO ET AL: "High pressure organic chemistry. XIV. A new powerful method for the transformation of lactams into.omega.-amino carboxamides under high pressure conditions" TETRAHEDRON LETT. (1992), 33(34), 4945-8, XP002171829
- A. HUBERT ET AL: "Carbon Dioxide Catalysis of the Formation of Pyroglutamic Acid" NATURE., vol. 182, no. 4630, 26 July 1958 (1958-07-26), page 259 XP002171830 MACMILLAN JOURNALS LTD. LONDON., GB ISSN: 0028-0836
- E. CESAROTTI ET AL: "Synthesis and Stereochemical Studies of the Chiral Ruthenium Complexes [Ru(n-C5H5){(S))dpompyr-PP'}X] [dpompyr = N-diphenylphosphino-2-(diphenylphosphinoxy methyl)pyrrolidone, X = H or Cl]. Crystal Structure of [(S)Ru(n-C5H5){(S)dpompyr-PP'}Cl]" JOURNAL OF THE CHEMICAL SOCIETY, DALTON TRANSACTIONS., no. 5, May 1987 (1987-05), pages 1149-1155, XP002171831 CHEMICAL SOCIETY. LETCHWORTH., GB ISSN: 1472-7773

## Description

The present invention relates to a novel process for the preparation of intermediates used for the preparation of contrast agents.

More particularly, the present invention relates to an improved process for the preparation of bile esters derivatives of general formula (I), 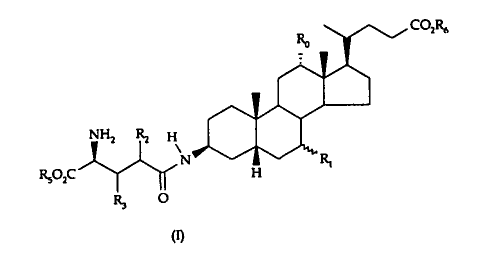 wherein;
R₀ is H or OH,
R₁ is H, α-OH or β-OH,
R₂ and R₃ are independently hydrogen, or straight or branched (C₁-C₂₀) alkyl optionally substituted with aryl,
R₅ is a straight or branched (C₁-C₄) alkyl and R₆ is a straight or branched (C₁-C₄) alkyl or a benzyl group.

The compounds of formula (I) are intermediates in the preparation of contrast agents, whose use in nuclear magnetic resonance diagnostics is extensively described in WO00/38738.

This latter document reports i.a. the synthesis of the compounds of formula (I), through a multistep process involving the transamidation of a N-protected-pyrrolidinone of formula (II), 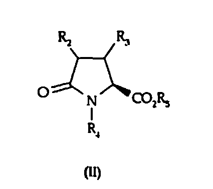 with an amine (III) H₂N-R* wherein R* is the reactive derivative of the convenient bile acid, to give an intermediate compound (IV) 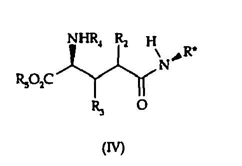 followed by the selective removal of the N-protecting group R₄.

The transamidation reaction maintains the stereochemistry at the chiral centre adjacent to the nitrogen atom of the starting pyrrolidinone and affords a secondary amide.

The selection of the R₄ protecting group is important because its cleavage should take place under conditions that do not affect the R₅ and R₆ groups. In the same patent application the use of a carbobenzyloxy (Cbz) protecting group for R₄, has been exemplified.

This prior art process, using a Cbz protecting group, presents however the following drawbacks which should be overcome for an industrial scale-up:
- the deprotection step involves the use of hydrogen and a catalyst;
- the intermediate N-Cbz protected compound is an oil which is not stored and, accordingly, is prepared just before use, thus rendering the overall industrial process much more complicated.

Protection of a pyrrolidinone nitrogen atom with a tert-butoxycarbonyl (Boc) group and reaction of the thus protected lactam with an amine has been described by H. Kotsuki et al. in Tetrahedron Letters, 33, No. 34, pp. 4945-4948, 1992. The results there reported show that in case of a N-Boc protected pyrrolidinone the reaction with an amine only proceeds when high pressures are employed, while the reaction with the same amine carried out under reflux but at atmospheric pressure, results in the complete recovery of the starting materials. The pressure values suggested in the above article are of the order of 10 kbar. These values might well be employed on a small, laboratory, scale but may create safety problems when used on a large, industrial, scale thus resulting to be practically unacceptable. In the same article, the Authors also report that in some cases sterically hindered amines did not react even at high pressures.

It has now been found that, contrary to what could be expected on the basis of the above article, it is possible to carry out the transamidation of a N-Boc-pyrrolidinone with an amine derived from a biliary acid under industrially acceptable conditions that do not require the use of high or anyway over atmospheric pressures.

It has furthermore been found that using the tert-butoxycarbonyl group as the N-protecting group in the synthesis of the compounds (I) above, it is possible to solve the technical problems of the prior art process. As a matter of fact selective cleavage of the tert-butoxycarbonyl group can be obtained under acidic conditions, thus avoiding the use of hydrogen, and the N-Boc protected pyrrolidinone esters are stable solid products that can be prepared in a separate step and stored without problems.

The present invention therefore relates to a process for the preparation of a compound of general formula (I), 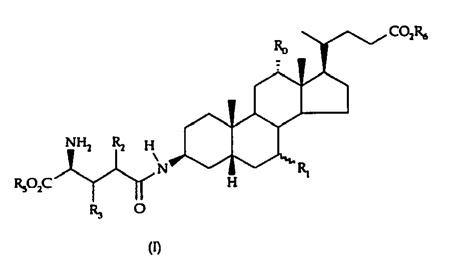 wherein;
R₀ is H or OH,
R₁ is H, α-OH or β-OH,
R₂ and R₃ are independently hydrogen, or straight or branched (C₁-C₂₀) alkyl optionally substituted with aryl,
R₅ is a straight or branched (C₁-C₄) alkyl and R₆ is a straight or branched (C₁-C₄) alkyl or a benzyl group,
which process comprises subjecting a compound of formula (II) 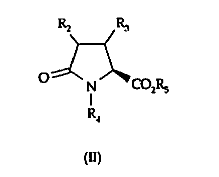 wherein R₂, R₃ and R₅ are as defined above and R₄ is tertbutoxycarbonyl to transamidation, by treatment with a compound of general formula (V) 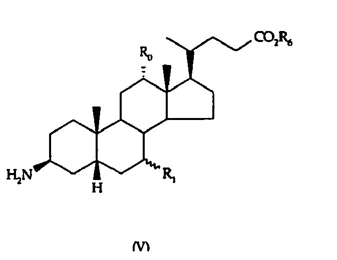 wherein R₀, R₁ and R₆ are as defined above, to give a compound of formula (VI): 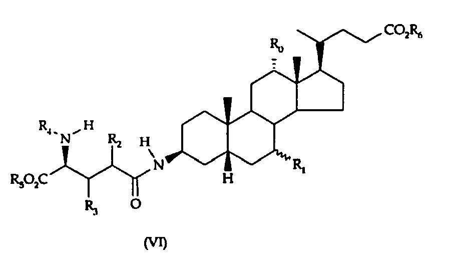 and selectively cleaving the R₄ protecting group under acid conditions.

In a preferred embodiment the present invention relates to a process for the manufacture of a compound of formula (I) wherein R₂ and R₃ are both hydrogen.

In a more preferred embodiment the present invention relates to a process for the manufacture of a compound of formula (I), wherein both R₂ and R₃ are hydrogen and R₅ is a straight (C₁-C₄) alkyl group, and even more preferably it is a methyl group.

In the above process the transamidation reaction of the first step is generally carried out by reacting one mole of the amine of formula (V), wherein R₀, R₁, and R₆ are as defined above, with from about 1 to about 1.5 mole of compound (II), wherein R₂, R₃, R₄, and R₅ are as defined above, in an organic solvent selected from the class of dipolar aprotic or apolar organic solvents. Suitable solvents are selected for instance from the group consisting of N,N-dimethylacetamide, N,N-dimethylformamide, ethyl acetate, butyl acetate, toluene, xylene, p-cymene, diethylbenzene and the like aromatic solvents where the aromatic ring bears one or more linear or branched (C₁-C₄) alkyl groups.

The reaction mixture is stirred at a temperature typically comprised between about 70 °C and about 130 °C, depending on the reactants and solvent employed.
As indicated above, the transamidation reaction does not require the use of high pressures as it easily proceeds at the atmospheric one.
Under these temperature and pressure conditions the transamidation reaction is complete generally in 12 to 30 hours.
The reaction mixture is then allowed to cool down to room temperature and the precipitate which forms is recovered by filtration, washed on filter and dried to yield the condensation product of formula (VI).

In the subsequent step the compound of formula (VI) is subjected to acid hydrolysis to remove the N-protecting group R₄ and give the final product of general formula (I).

The reaction preferably consists in a slow addition of an inorganic or organic acid, under anhydrous (e.g. gas) or aqueous form, to a solution of the compound (VI) in a (C₁-C₄)alkanol, such as methanol or ethanol, or an inert organic solvent such as tetrahydrofuran, dioxane, and the like solvents, while maintaining a reaction temperature from 15 to 60 °C.

The resulting solution is kept at this temperature until removal of the R₄ group is complete. Depending on the acid used in the conversion of (VI) to (I) and the temperature this will take from 0.5 to 20 hours.

In this step the acid compound is preferably selected from: HCl gas, HCl gas in MeOH, HCl in MeOH, HBr in CH₃CO₂H, aq. H₂SO₄, CF₃CO₂H, CH₃CO₂H, oxalic acid, methanesulfonic acid and p-toluenesulfonic acid.

The acid is added in a quantity corresponding to 1÷3 moles per mole of (VI).

The obtained compound of formula (I) is isolated either as a salt of the acid used to cleave the protecting group or as a free amine of general formula (I).

The isolation of (I) as a free amine, is carried out by first neutralising the acidic mixture obtained at the end of reaction, by the addition of a base preferably selected from tertiary amines such as triethylamine or diisopropylethylamine.

The use of a hindered tertiary amine, affords the isolation of compounds (I) minimizing the possible formation of by products due to secondary reactions, e.g. hydrolysis of the ester groups or transamidation. In fact aqueous bases, like for example aq. NaOH or KOH, can hydrolyse the ester groups, and in particular the ester group present on the glutamic chain which is easier to cleave than the ester group in the cholanoic moiety. Furthermore, the use of a NH₃ solution or of a primary or secondary amine can promote a transamidation reaction.

By using a tertiary amine the compound of formula (I) is isolated in this step in a yield ranging from 80 to 97%.

The starting compounds of formula (V), are prepared according to what is disclosed in WO-A-95/32741 or in PCT/EP00/08226. These compounds are esters of bile acids in which a β- amino group is always present in position 3, replacing the hydroxy group.

The most important examples of bile acids of the present invention are selected from the group consisting of cholic, chenodeoxycholic, deoxycholic, ursodeoxycholic, and lithocholic acids represented by the following formulae. 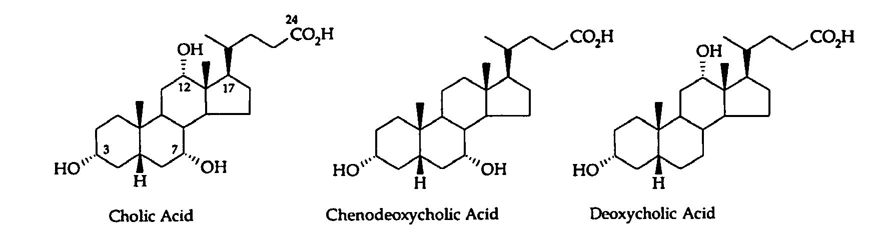 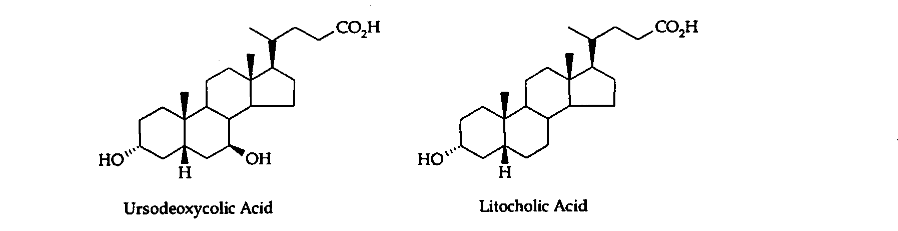 The compounds of formula (II) can be prepared from the corresponding 5-oxoproline derivative (VII) 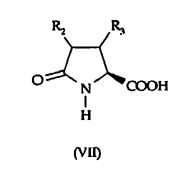 by first esterification of the carboxy group by reaction with the suitably selected (C₁-C₄)alkanol by *per se* known methods, followed by introduction of the N-protecting group R₄.

General methods for the protection of the 5-oxoproline esters can be derived from the following references that describes protection of the methyl ester with the tert-butoxycarbonyl group: JP05247047; Eur. J. Org. Chem., 1999, 1581-1584; Tetrahedron Lett., 1998, 39, 4789-4792; Tetrahedron Lett., 1993, 34, 5455-5458; Chem. Pharm. Bull., 1991, 39, 1199-1212; J. Org. Chem. 1983, 48, 2424-2426.

Alternatively and preferably the compounds of formula (II) are obtained by esterification of an L-glutamic acid derivative of formula (VIII), preferably in the form of an addition salt with a mineral acid, e.g. the hydrochloride, 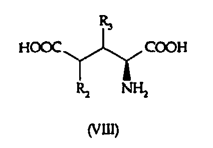 wherein R₂ and R₃ are as defined above to give the corresponding di-(C₁-C₄)alkyl ester (IX), 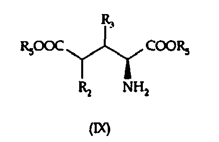 preferably in the form of an addition salt with a mineral acid e.g. the hydrocloride, followed by cyclization of the above diester to yield the 5-oxoproline (C₁-C₄)alkyl ester (X) 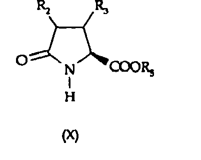 and introduction of the suitably selected N-protecting group R₄ to afford the compound of formula (II).

In particular, esterification of the compound o formula (VIII) is carried out by first suspending the compound in the suitably selected (C₁-C₄) alkanol and then adding at least 2 moles of SOCl₂ per mole of (VIII). The temperature is maintained at 0-5 °C during the addition, and then the reaction is completed in almost one day at room temperature. The solvent is evaporated and the thus obtained product is directly used, without any purification, in the next step. In this step the acid addition salt of the diester (IX) is neutralized with KOH in a (C₁-C₄) alkanol solution, and the precipitated KCl is filtered off. The filtrate is evaporated and then heated for few (1 to 7 ) hours, at a temperature varying from 80 to 130 °C to give the cyclized product to be used directly in the final step of introduction of the R₄ protecting group.

Finally protection of the lactam nitrogen of (X) with the R₄ protecting group is carried out according to classical methods of protection described in the literature. This can be easily achieved by reacting the compound of formula (X) with at least the equimolar amount of the corresponding compound (XI)

R₄-X (XI)

wherein, when R₄ is a tert-butoxycarbonyl group, X is a tert-butoxy group so that the reaction is carried out with the corresponding carbonate. R₄ is a tert-butoxycarbonyl group, and the introduction of this protecting group is carried out using the ditertbutyl carbonate, the reaction is preferably carried out adding at least the equimolar amount of the ditertbutyl carbonate to a solution containing one mole of the compound of formula (X) in the presence of a solvent selected from the classes of polar aprotic and apolar organic solvents, such as C₁-C₄ alkyl esters of acetic acid, acetonitrile, aromatic solvents such as toluene, xylene and the like solvents. Ethyl acetate and acetonitrile are the preferred ones. The reaction is normally catalyzed by the addition of, for example, 4-(dimethylamino)pyridine, in a quantity ranging from 0.01 to 0.1 moles per mole of (X).

The reaction is typically completed in a couple of hours at room temperature and the crude residue obtained upon evaporation of the solvent, is purified by washing and by crystallization from a solvent, preferably EtOAc and/or n-hexane.

The following examples further illustrate the process according to the present invention in one of its preferred embodiments. They should not be interpreted anyway as a limitation to the scope of the invention.
The TLC and HPLC methods reported in the following Examples are carried out as indicated below.

### Thin Layer Chromatography:

Silica gel platens used for the TLC are: 60 F₂₅₄
Eluent A : 70 : 25 : 3 CHCl₃/MeOH/25% NH₄OH
Eluent B : 80 : 20 EtOAc/CH₂Cl₂
Detection : exposure to Cl₂ vapours + o-tolidine

### Analytical HPLC methods:

### Method A

- Stationary phase:: Chiralcel OD-H, 250 x 4.6 mm column packed by Daicel
- Temperature:: 40 °C
- Mobile phase:: isocratic elution: A/B = 93 : 7
A = *n*-hexane, B = ethanol
- Flow rate:: 1.0 mL min⁻¹
- Detection (UV):: 210 nm
- Injection:: 20 µL
- Sample concentration:: 2.0 mg mL⁻¹ (racemic mixture), 5.0 mg mL⁻¹ (optically active)

### Method B

Stationary phase: Lichrosorb RP-Select B 5 µm, 250 x 4 mm column packed by Merck KGaA
Temperature: 45°C
Mobile phase: gradient elution, A = 0.017 M H₃PO₄ in water, B = CH₃CN
Gradient timetable:

| min | %A | %B |
|---|---|---|
| 0 | 82 | 18 |
| 30 | 15 | 85 |
| 45 | 15 | 85 |

Flow rate: 1 mL min⁻¹
Detection (UV): 210 nm
Injection: 10 µL
Sample concentration: 2 mg mL⁻¹

### Method C

- Stationary phase:: Chiralcel OD-H, 250 x 4.6 mm column packed by Daicel
- Temperature:: 40 °C
- Mobile phase:: isocratic elution: A/B = 95 : 5
A = *n*-hexane, B = ethanol
- Flow rate:: 1.0 mL min⁻¹
- Detection (UV):: 210 nm
- Injection:: 20 µL
- Sample concentration:: 0.4 mg mL⁻¹ (racemic mixture), 1.0 mg mL⁻¹ (optically active)

### Method D

- Stationary phase:: Chiralcel OD-H; 250 x 4.6 mm column packed by Daicel;
- Temperature:: 40 °C;
- Mobile phase:: isocratic elution: A/B = 92:8;
A = *n*-hexane, B = ethanol
- Flow rate:: 1.0 mL min⁻¹;
- Detection (UV):: 210 nm;
- Injection:: 10 µL;
- Sample concentration:: 2.0 mg mL⁻¹ (racemic mixture), 5.0 mg mL⁻¹ (optically active);

### Method E

Stationary phase: Lichrosorb RP-Select B 5 µm; 250 x 4 mm column packed by Merck KGaA;
Temperature: 45°C;
Mobile phase: gradient elution;
A = 0.017 M H₃PO₄ in water, B = CH₃CN
Gradient timetable:

| min | %A | %B |
|---|---|---|
| 0 | 82 | 18 |
| 30 | 15 | 85 |
| 45 | 15 | 85 |

Flow rate: 1 mL min⁻¹;
Detection (UV): 210 nm;
Injection: 10 µL;
Sample concentration: 1 mg mL⁻¹

### Method F

- Stationary phase:: Chiralcel OD; 250 x 4.6 mm column packed by Daicel;
- Temperature:: 40 °C;
- Mobile phase:: isocratic elution: A/B = 85:15;
A = *n*-hexane, B = 2-propanol
- Flow rate:: 1.0 mL min⁻¹;
- Detection (UV):: 210 nm;
- Injection:: 20 µL;
- Sample concentration:: 0.7 mg mL⁻¹ (racemic mixture), 3.0 mg mL⁻¹ (optically active);

### Example 1

### a) Preparation of (3β,5β,12α)-12-hydroxy-3-[[5-methoxy-1,5-dioxo-4(S)-4-[[(1,1-dimethylethoxy)carbonyl]amino]pentyl]amino]cholan-24-oic acid methyl ester (II: R₅= Me, R₂=R₃=H, R₄=t-butoxycarbonyl; V: R₀=OH, R₁= H, R₆=Me; VI: R₀=OH, R₁=H, R₂=R₃=H, R₄=t-butoxycarbonyl, R₅=Me, R₆=Me)

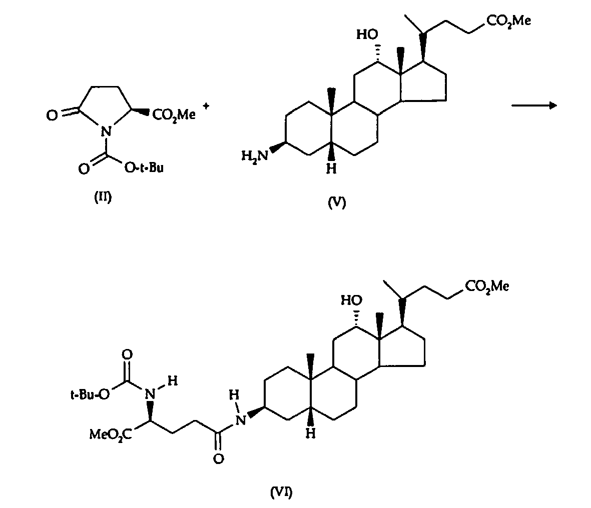

A suspension of (V) (625 g; 1.54 mol) and (II) (374.6 g; 1.54 mol) in toluene (1.54 L) was stirred at 90 °C for 24 h. The reaction mixture was then allowed to cool to room temperature overnight, the precipitate was recovered by filtration, washed with toluene and dried (40 °C; 2 kPa) to afford (VI) as a 1:1 clathrate with toluene (889.2 g; 1.2 mol).
Yield 78 %.
HPLC (method D): e.e.> 99.6 %
HPLC (method E): 98 %
The ¹H-NMR, ¹³C-NMR, IR and MS spectra are consistent with the indicated structure.

### b) Preparation of (3β,5β,12α)-3-[[4(S)-4-amino-5-methoxy-1,5-dioxopentyl]amino]-12-hydroxycholan-24-oic acid methyl ester (I: R₀=OH, R₁=H, R₂=R₃=H, R₅=R₆=Me)

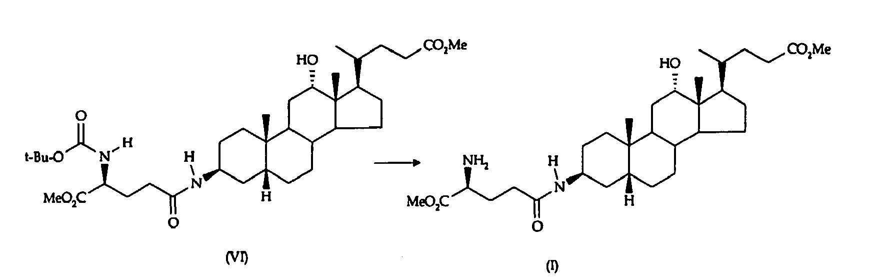

To a solution of the compound obtained in step a) as a 1:1 clathrate with toluene (231.6 g; 0.31 mol), in MeOH (1.16 L), methanesulfonic acid (56.7 g; 0.6 mol) was slowly added while maintaining the reaction temperature below 20 °C. The resulting solution was stirred at r.t. for 24 h. Then diisopropylethylamine (77.6 g; 0.6 mol) was added and the solution was evaporated to a crude residue that was taken up with water. After one hour stirring at r.t. the solid was filtered, washed with water and dried to afford the compound of the title (149.1 g; 0.27 mol) as a white solid. Yield 87 %.
HPLC (method E): 98.4 % (area %)
HPLC (method F): e.e.> 99.5%
The ¹H-NMR, ¹³C-NMR, IR and MS spectra are consistent with the indicated structure.

### Example 2

### Preparation and isolation of (3β,5β,12α)-3-[[4(5)-4-amino-5-methoxy-1,5-dioxopentyl]amino]-12-hydroxycholan-24-oic acid methyl ester dihydrocloride

A solution of the compound obtained in Example 1 a) as the 1:1 clathrate with toluene (30 g; 40 mmol), in 2.5 M HCl in MeOH (100 mL) was stirred at r.t. for 15 h then the solution was seeded. After 2 h at 0 °C, the solid was filtered, washed with cold 1.5 M HCl in MeOH (30 mL) and dried to obtain the compound of the title (21.4 g; 34.4 mmol) containing a further mole of HCl as a white solid.
Yield 86 %.
TLC: (eluent B) Rf 0.68
HPLC (method E) : 96.9 % (area %)
HPLC (method F) : e.e.> 99.5 %
Argentometric titer (0.1 N AgNO₃): 97.3 %
The ¹H-NMR, ¹³C-NMR, IR and MS spectra are consistent with the indicated structure.

### Example 3

### Preparation of the starting (S)-5-oxo-1,2-pyrrolidinedicarboxylic acid 1-(1,1-dimethylethyl)2-methyl ester

### a) Preparation of L-glutamic acid dimethyl ester hydrochloride (IX; R₅= -Me, R₂=R₃=H)

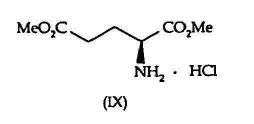

SOCl₂ (732 g; 6.15 mol) was added over 2 h to a suspension of either L-glutamic acid hydrochloride (VIII; R₂=R₃=H) (551 g; 3 mol) or L-glutamic acid (441.4 g; 3 mol) in MeOH (3.5 L) stirred at 0-5 °C. After about 3.5 h at r.t. the reaction mixture turned into a clear solution that was stirred for 20 h. The solvent was evaporated to give the above compound (650.8 g) as a thick oil that was used in the following step without any purification.
TLC : Rf 0.79 (Eluent A)
Argentometric titer (0.1 N AgNO₃): 105.3 %
The ¹H-NMR, ¹³C-NMR, and MS spectra are consistent with the indicated structure.

### b) Preparation of L-5-oxoproline methyl ester (X; R₂=R₃=H, R₅=Me)

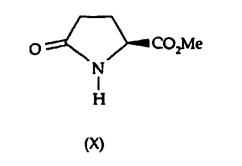

3 M KOH in MeOH (1.08 L; 3.24 mol) was added over 0.5 h to a solution of the compound obtained in step a) above (650.6 g), in MeOH (1 L) causing the precipitation of KCl that was filtered off. The clear solution was concentrated, filtered (as the precipitation of further KCl occurred) and evaporated. The residue was heated at 115 °C at atmospheric pressure for about 1 h while distilling MeOH produced by the cyclization reaction to obtain a crude product (447 g) as a colourless oil which was used in the next step without purification.
TLC : Rf 0.71 (Eluent A)
HPLC : S/R ratio 99.0 : 1.0 (Method A)
The ¹H-NMR, ¹³C-NMR, and MS spectra are consistent with the indicated structure.

### c) Preparation of (S)-5-oxo-1,2-pyrrolidinedicarboxylic acid 1-(1,1-dimethylethyl) 2-methyl ester (II; R₂=R₃=H; R₅=Me, R₄=-COO-t-Bu)

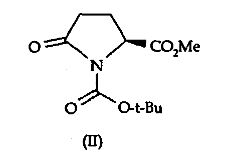

Di-*t*-butyl dicarbonate (611 g; 2.8 mol) was added over 1 h to a cloudy solution of the compound obtained in above step b) (447 g), and 4-(dimethylamino)pyridine (6.1 g; 0.05 mol) in acetonitrile (2.8 L) stirred at 15-18 °C. After 2 h the solvent was evaporated. The residue was dissolved in EtOAc, washed with aq. pH 5.7 phosphate buffer and H₂O. After drying, the solvent was evaporated to give the compound of the title as a thick oil. The latter was dissolved in EtOAc and the solution slowly diluted with n-hexane to induce crystallisation. After 15 h at r.t. the solid was filtered, washed with n-hexane and dried to afford the compound of the title (474.3 g; 1.95 mol) as a white solid.
Yield 65 %. The mother liquors and the washings were combined and evaporated. The oily residue was treated with n-hexane to give a second crop of the compound of the title (73 g; 0.3 mol) (yield 10 %) as a whitish solid of purity similar to the one of the first crop. Overall yield from glutamic acid 75 %.
mp : 70-71.5 °C
TLC : Rf 0.74 (Eluent B)
HPLC (method B): first crop 99.6 % (area %)
second crop 98.8 % (area %)
HPLC (method C): S/R ratio first crop 100 : 0
second crop 100 : 0
The ¹H-NMR, ¹³C-NMR, IR and MS spectra are consistent with the indicated structure.
The compound thus obtained can then be used directly in the process of the invention

## Claims

1. A process for the preparation of a bile ester derivative of general formula (I), 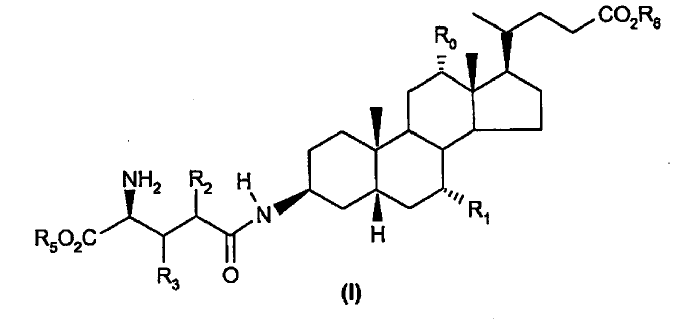 wherein:
R₀ is H or OH,
R₁ is H, α-OH or β-OH,
R₂ and R₃ are independently hydrogen, or straight or branched (C₁-C₂₀) alkyl optionally substituted with aryl,
R₅ is a straight or branched (C₁-C₄) alkyl and
R₆ is a straight or branched (C₁-C₄) alkyl or a benzyl group,
which process comprises subjecting a compound of formula (II), 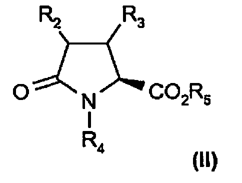 wherein R₂, R₃ and R₅ are as defined above and R₄ is tert-butoxycarbonyl, to transamidation, by treatment with a compound of general formula (V), 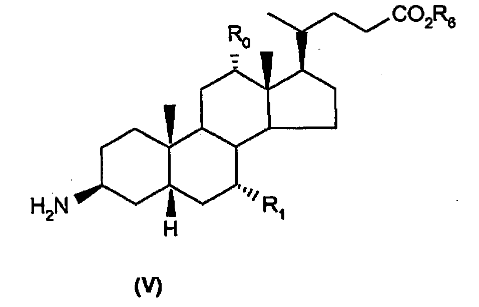 wherein R₀, R₁ and R₆ are as defined above,
to give a compound of formula (VI) 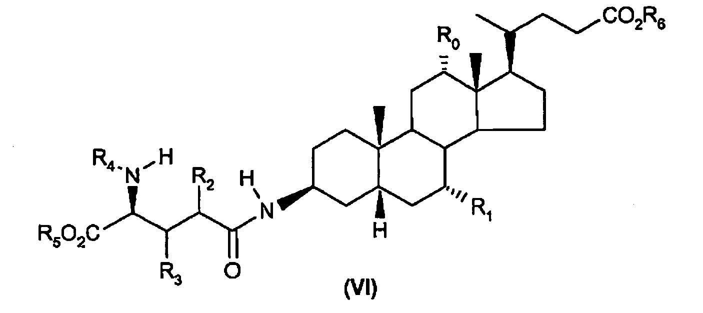 and selectively cleaving the R₄ protecting group under acid conditions.

2. The process of claim 1, for the preparation of a compound of formula (I) wherein R₂ and R₃ are hydrogen.

3. The process of claim 2, for the preparation of a compound of formula (I) wherein R₅ is a methyl group.

4. The process of any of the preceding claims wherein the transamidation is carried out at atmospheric pressure.

5. The process of any of the preceding claims wherein the transamidation is carried out using one mole of compound (V) per 1 to 1.5 mole of compound (II), in a solvent selected from the dipolar aprotic and apolar organic solvents at a temperature from 70 to 130°C.

6. The process of any of the preceding claims wherein selective cleavage of the protective group R₄ is carried out using 1 to 3 mole of acid per mole of (II), where the acid is selected from HCl gas, HCl gas in MeOH, HCl in MeOH, HBr in CH₃CO₂H, aq. HCl, aq. H₂SO₄, CF₃CO₂H, CH₃CO₂H, oxalic acid, methanesulfonic acid and p-toluenesulfonic acid.

7. The process of any of the preceding claims wherein the end compound of formula (I) is isolated as the free amine by addition of a tertiary amine, selected from triethylamine or diisopropylethylamine.

8. The process of any of the preceding claims for the preparation of (3β,5β,12α)-3-[[4(S)-4-amino-5-methoxy-1,5-dioxopentyl]amino]-12-hydrocholan-24-oic acid methyl ester of formula

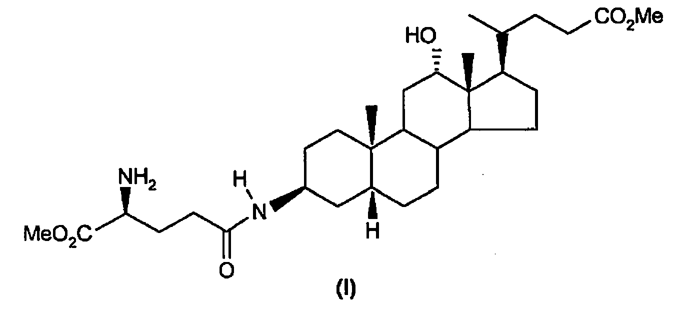

9. The process of any of the preceding claims wherein the starting compound of formula (II) is prepared through esterification of the corresponding glutamic acid derivative of formula (VIII), 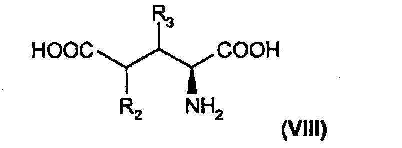 preferably in the form of an addition salt with a mineral acid, to give the di-(C₁-C₄)alkyl ester (IX), 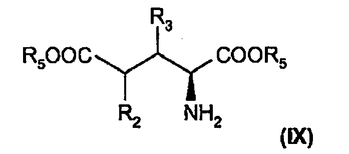 preferably in the form of an addition salt with a mineral acid, followed by cyclization of said diester to yield the corresponding 5-oxo-proline(C₁-C₄) alkyl ester (X) 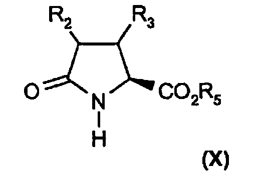 and introduction of the group R₄.

## Patentansprüche

1. Verfahren zur Herstellung eines Gallensäureesterderivats mit der allgemeinen Formel (I) 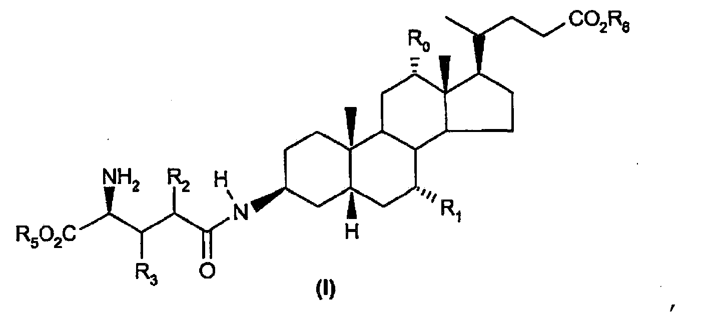 wobei
R₀ H oder OH ist,
R₁ H, α-OH oder β-OH ist,
R₂ und R₃ unabhängig Wasserstoff oder geradkettiges oder verzweigtes (C₁-C₂₀)-Alkyl sind, das gegebenenfalls mit Aryl substituiert ist,
R₅ ein geradkettiges oder verzweigtes (C₁-C₄)-Alkyl ist, und
R₆ ein geradkettige oder verzweigte (C₁-C₄)-Alkyl oder eine Benzylgruppe ist,
und bei dem man eine Verbindung mit der Formel (II) 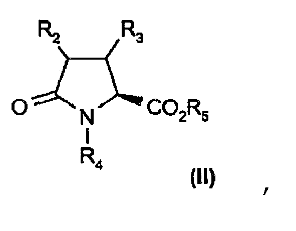 in der R₂, R₃ und R₅ wie oben definiert sind und R₄ tert.-Butoxycarbonyl ist, der Transamidierung durch Behandlung mit einer Verbindung mit der allgemeinen Formel (V) 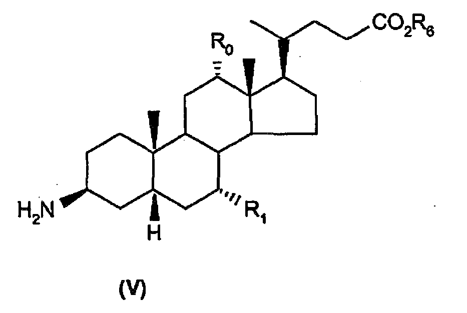 unterzieht, wobei R₀, R₁ und R₆ wie bereits definiert sind,
um eine Verbindung mit der Formel (VI) 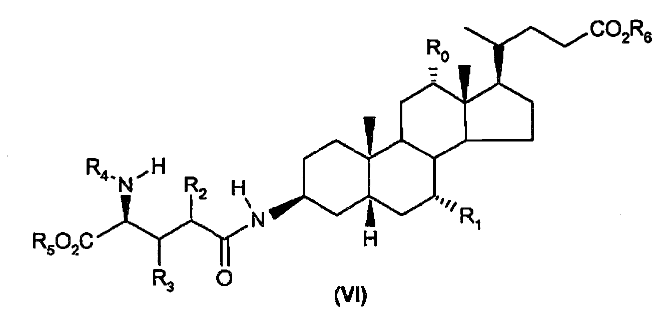 zu erhalten, und man die R₄-Schutzgruppe unter Säurebedingungen selektiv abspaltet.

2. Verfahren nach Anspruch 1 zur Herstellung einer Verbindung mit der Formel (I), in der R₂ und R₃ Wasserstoff sind.

3. Verfahren nach Anspruch 2 zur Herstellung einer Verbindung mit der Formel (I), in der R₅ eine Methylgruppe ist.

4. Verfahren nach einem der vorhergehenden Ansprüche, bei dem die Transamidierung bei atmosphärischem Druck durchgeführt wird.

5. Verfahren nach einem der vorhergehenden Ansprüche, bei dem die Transamidierung unter Verwendung von einem Mol der Verbindung (V) pro 1 bis 1,5 Mol der Verbindung (II) in einem Lösungsmittel bei einer Temperatur von 70 bis 130°C durchgeführt wird, das aus dipolar-aprotischen und apolaren organischen Lösungsmitteln ausgewählt ist.

6. Verfahren nach einem der vorhergehenden Ansprüche, bei dem die selektive Abspaltung der Schutzgruppe R₄ unter Verwendung von 1 bis 3 Mol Säure pro Mol (II) durchgeführt wird, wobei die Säure ausgewählt ist aus HCl-Gas, HCl-Gas in MeOH, HCl in MeOH, HBr in CH₃CO₂H, wässr. HCl, wässr. H₂SO₄, CF₃CO₂H, CH₃CO₂H, Oxalsäure, Methansulfonsäure und p-Toluolsulfonsäure.

7. Verfahren nach einem der vorhergehenden Ansprüche, bei dem die Endverbindung mit der Formel (I) als freies Amin durch Zugabe von tertiärem Amin ausgewählt aus Triethylamin oder Diisopropylethylamin isoliert wird.

8. Verfahren nach einem der vorhergehenden Ansprüche zur Herstellung von (3β,5β,12α)-3-[[4(S)-4-Amino-5-methoxy-1,5-dioxopentyl]amino]-12-hydrocholan-24-säuremethylester mit der Formel:

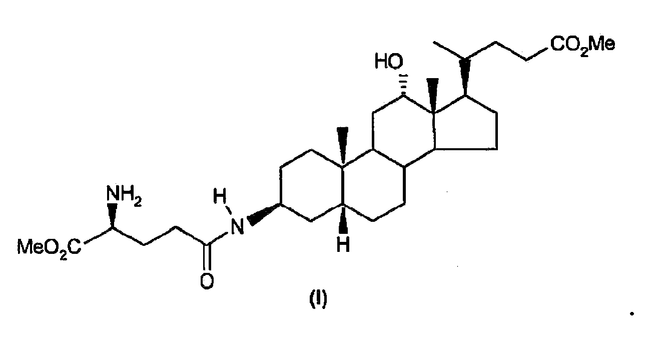

9. Verfahren nach einem der vorhergehenden Ansprüche, bei dem man die Ausgangsverbindung mit der Formel (II) herstellt, in dem man das entsprechende Glutaminsäurederivat mit der Formel (VIII) 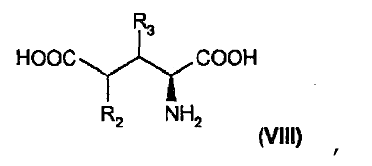 vorzugsweise in Form eines Additionssalzes mit einer Mineralsäure, verestert, um den Di-(C₁ bis C₄)-alkylester (IX) 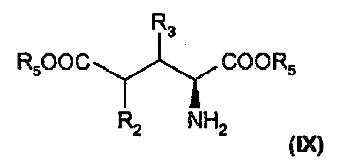 vorzugsweise in Form eines Additionssalzes mit Mineralsäure zu erhalten, gefolgt von der Cyclisierung des Diesters, um den entsprechenden 5-Oxoprolin-(C₁-C₄)-alkylester (X) 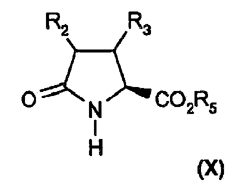 zu erhalten, und Einführung der Gruppe R₄.

## Revendications

1. Procédé de préparation d'un dérivé ester d'acide biliaire, de formule générale (I) : 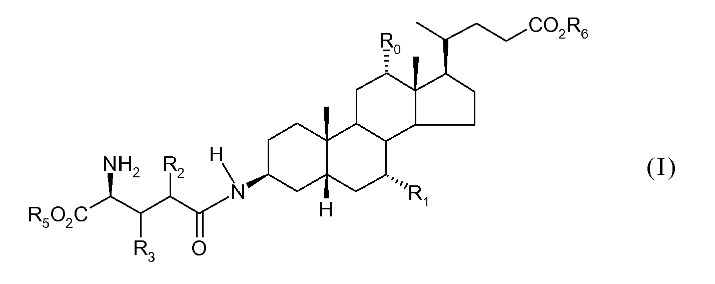 dans laquelle :
- R₀ représente un atome d'hydrogène ou un groupe hydroxyle,
- R₁ représente un atome d'hydrogène ou un groupe α-hydroxyle ou β-hydroxyle,
- R₂ et R₃ représentent chacun, indépendamment, un atome d'hydrogène ou un groupe alkyle en C₁₋₂₀ à chaîne droite ou ramifiée, éventuellement porteur d'un substituant aryle ;
- R₅ représente un groupe alkyle en C₁₋₄ à chaîne droite ou ramifiée ;
- et R₆ représente un groupe alkyle en C₁₋₄ à chaîne droite ou ramifiée, ou un groupe benzyle ;
lequel procédé comporte le fait de soumettre un composé de formule (II) : 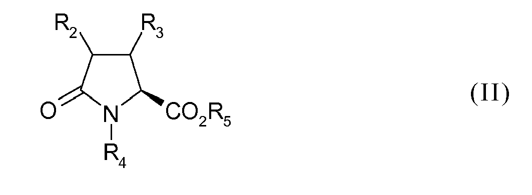 dans laquelle R₂, R₃ et R₅ ont les significations indiquées ci-dessus et R₄ représente un groupe tertiobutoxy-carbonyle,
à une trans-amidation, par traitement avec un composé de formule générale (V) : 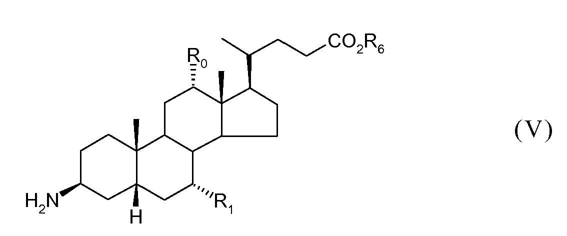 dans laquelle R₀, R₁ et R₆ ont les significations indiquées ci-dessus, de manière à obtenir un composé de formule (VI) : 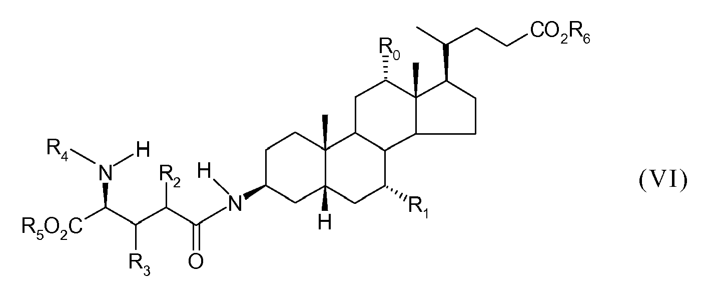 et le fait d'éliminer sélectivement, en milieu acide, le groupe protecteur symbolisé par R₄.

2. Procédé, conforme à la revendication 1, de préparation d'un composé de formule (I) dans laquelle R₂ et R₃ représentent des atomes d'hydrogène.

3. Procédé, conforme à la revendication 2, de préparation d'un composé de formule (I) dans laquelle R₅ représente un groupe méthyle.

4. Procédé conforme à l'une des revendications précédentes, dans lequel on effectue la réaction de trans-amidation sous pression atmosphérique.

5. Procédé conforme à l'une des revendications précédentes, dans lequel on effectue la réaction de trans-amidation en utilisant 1 mole de composé de formule (V) pour 1 à 1,5 mole de composé de formule (II), dans un solvant choisi parmi les solvants organiques apolaires ou dipolaires aprotiques, à une température de 70 à 130 °C.

6. Procédé conforme à l'une des revendications précédentes, dans lequel on effectue la réaction d'élimination sélective du groupe protecteur symbolisé par R₄ en employant 1 à 3 moles d'acide par mole de composé de formule (II), l'acide étant choisi parmi les suivants : chlorure d'hydrogène gazeux, chlorure d'hydrogène gazeux dans du méthanol, chlorure d'hydrogène dans du méthanol, bromure d'hydrogène dans de l'acide acétique, chlorure d'hydrogène en solution aqueuse, acide sulfurique en solution aqueuse, acide trifluoroacétique, acide acétique, acide oxalique, acide méthane-sulfonique et acide para-toluène-sulfonique.

7. Procédé conforme à l'une des revendications précédentes, dans lequel on isole le composé final de formule (I) à l'état d'amine libre, par addition d'une amine tertiaire choisie parmi de la triéthylamine et de la diisopropyl-éthylamine.

8. Procédé, conforme à l'une des revendications précédentes, de préparation de l'ester méthylique de l'acide (3β,5β,12α)-3-[(4(S)-4-amino-5-méthoxy-1,5-dioxo-pentyl)amino]-12-hydroxy-cholane-24-oïque, de formule :

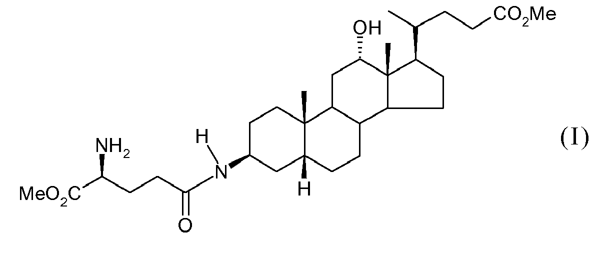

9. Procédé conforme à l'une des revendications précédentes, pour lequel on a préparé le composé de départ de formule (II) en estérifiant un dérivé correspondant de l'acide glutamique, de formule (VIII) : 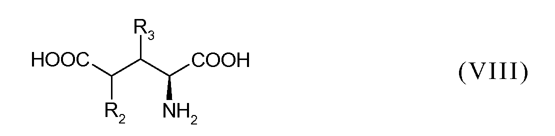 utilisé de préférence à l'état de sel d'addition d'un acide minéral, de manière à obtenir un ester de dialkyle en C₁₋₄, de formule (IX) : 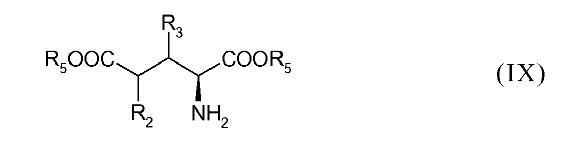 de préférence à l'état de sel d'addition d'un acide minéral, et en effectuant ensuite la cyclisation de ce diester, de manière à obtenir l'ester d'alkyle en C₁₋₄ de 5-oxo-proline correspondant, de formule (X) : 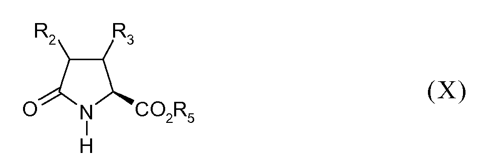 et l'introduction du groupe symbolisé par R₄.
